# EUROPEAN PATENT SPECIFICATION

(11) **EP 4 126 051 B1**
(45) Date of publication and mention of the grant of the patent: **12.11.2025**
(21) Application number: 21715649.6
(22) Date of filing: 01.04.2021
(51) Int. Cl.: A61K 39/395, A61K 45/06, A61K 39/00, C07K 16/28, A61P 35/00

(54) **REDUCED CALORIC INTAKE AND IMMUNOTHERAPY FOR THE TREATMENT OF CANCER**
REDUZIERTE KALORIENAUFNAHME UND IMMUNTHERAPIE ZUR BEHANDLUNG VON KREBS
APPORT CALORIQUE RÉDUIT ET IMMUNOTHÉRAPIE POUR LE TRAITEMENT DU CANCER

(30) Priority: 03.04.2020 IT 202000007153
(43) Date of publication of application: 08.02.2023
(73) Proprietor: IFOM - Istituto Fondazione Di Oncologia Molecolare ETS In Breve IFOM ETS, 20139 Milano (IT)
(72) Inventor: LONGO, Valter, 20139 Milano (MI) (IT); CORTELLINO, Salvatore, 20139 Milano (MI) (IT)
(74) Representative: Pace Napoleone, Maria
(86) International application number: PCT/EP2021/058656
(87) International publication number: WO 2021/198439

(56) References cited:
- WO-A1-2017/050849
- WO-A1-2019/175113
- ANONYMOUS: "Fasting-mimicking Diet in Patients Undergoing Active Cancer Treatment - Full Text View - ClinicalTrials.gov", 2 August 2018 (2018-08-02), XP055753298, Retrieved from the Internet <URL:https://clinicaltrials.gov/ct2/show/NCT03595540> [retrieved on 20201124]
- SARAH LÉVESQUE ET AL: "A synergistic triad of chemotherapy, immune checkpoint inhibitors, and caloric restriction mimetics eradicates tumors in mice", ONCOIMMUNOLOGY, vol. 8, no. 11, 7 September 2019 (2019-09-07), pages e1657375, XP055752965, DOI: 10.1080/2162402X.2019.1657375
- DANIEL AJONA ET AL: "Short-term starvation reduces IGF-1 levels to sensitize lung tumors to PD-1 immune checkpoint blockade", NATURE CANCER, vol. 1, no. 1, 1 January 2020 (2020-01-01), pages 75 - 85, XP055753021, DOI: 10.1038/s43018-019-0007-9
- NENCIONI ALESSIO ET AL: "Fasting and cancer: molecular mechanisms and clinical application", NATURE REVIEWS CANCER, NATURE PUB. GROUP, LONDON, vol. 18, no. 11, 16 October 2018 (2018-10-16), pages 707 - 719, XP036668994, ISSN: 1474-175X, [retrieved on 20181016], DOI: 10.1038/S41568-018-0061-0
- DI BIASE STEFANO ET AL: "Fasting-Mimicking Diet Reduces HO-1 to Promote T Cell-Mediated Tumor Cytotoxicity", CANCER CELL, CELL PRESS, US, vol. 30, no. 1, 11 July 2016 (2016-07-11), pages 136 - 146, XP029636921, ISSN: 1535-6108, DOI: 10.1016/J.CCELL.2016.06.005

## Description

### FIELD OF THE INVENTION

The present invention relates to at least one reduced caloric intake cycle and at least one immunotherapeutic agent for use in the treatment of cancer. In particular, the agent is a PDL-1 inhibitor, namely an anti-PDL1 antibody.

### BACKGROUND OF THE INVENTION

Tumours develop different strategies to evade the immune response in order to promote growth and metastasis. Tumour hijacks immune checkpoint pathways, developed to limit inflammatory and immune responses, by expressing programmed death-ligand 1 (PD-L1) on their cells surface, and by reshaping the tumour immune microenvironment in order to protect itself from the immune cell mediated T cell cytotoxicity.

Tumour immunogenicity varies between cancer, however high level of tumour infiltrating lymphocytes (TILs) is associated with a better prognosis in cancer patient (Foley EJ, 1953; Smith HJ, 1966). In cancer the immune checkpoint activation and the immunosuppressive microenvironment render TILs dysfunctional and exhausted. TILs express multiple co-inhibitory or checkpoint receptors, such as cytotoxic T lymphocyte antigen-4 (CTLA-4), programmed cell death-1 (PD-1), T cell immunoglobulin and mucin-domain containing-3 (TIM-3), and lymphocyte-activation gene (LAG-3) (Walunas TL et al., 1994; Fourcade J et al., 2010; Matsuzaki Jet al.,2010). The blockade of these checkpoint reverse TILs exhaustion and improve cytotoxicity and proliferation of these cells.

Immune checkpoint inhibitors (ICI) therapy, including antibodies against PD-1, PD-L1 and CTLA-4, achieve substantial and durable response across many tumour types. However only a subset of cancer patients responds to ICI therapies. For instance, the highest response rates to single-agent PD-1 blockade therapy occur in Hodgkin's lymphoma with a range between 80 to 90%, whereas in melanoma patients the response rate is between 35 to 40%. In NSCLC and head and neck, gastroesophageal, bladder and urothelial cancers, hepatocellular carcinoma and renal cell carcinoma the response rates ranges from 15 to 25% (Topalian SL et al., 2012; Garon EB et al., 2015; Motzer RJ et al., 2015; Rosenberg JE et al., 2016; Wolchok JD et al., 2017; El-Short-term starvation is demonstrated to synergize with PD-1 blockade to inhibit lung cancer progression and metastasis (D. Ajona et al, Nature Cancer, Vol. 1, no. 1, January 2020, pages 75-85).Khoueiry AB et al., 2017; Kim ST et al., 2017; Ribas A & Wolchok JD, 2018). ADD PAGE 1A CTLA-4 monotherapy produces durable responses in 22% of melanoma patients (Schadendorf D et al., 2015), whereas the response rate is below 10% in other solid tumors including pancreatic adenocarcinoma, renal cell carcinoma (Yang JC, et al.,2007), B-cell lymphoma (Lesokhin AM Lesokhin AM et al., 2016), prostate cancer (Slovin SF et al., 2013), refractory colorectal cancer, hepatocellular carcinoma (Sangro B et al., 2013), and malignant mesothelioma (Calabro L et al., 2013).

The anti-CTLA-4 and anti-PD-1 combined treatment achieve high response rate of 58% in melanoma patients (but was associated with severe toxicity (Larkin J et al., 2015; Postow MA, 1015). Ongoing clinical trials are testing the efficacy of PD-1 and CTLA-4 in patients affected by renal cell carcinoma and MSI-high colorectal cancer (Sade-Feldman M. et al., 2017).

The most common adverse event encountered for PD-1/PD-L1 and CTLA-4 pathway inhibitor are fatigue, diarrhoea, rash, and pruritus in 15 to 20% of patients (Robert C et al., 2015; Garon EB et al., 2015; Ribas A et al., 2016; Rosenberg JE et al., 2016; Robert C et al, 2015), whereas smaller percentage of patients develop endocrinopathies , such as thyroid disorders (10 to 15%), hypophysitis, adrenal gland disorders (1 to 3%), and type 1 diabetes (1%), or visceral organ inflammatory toxicities (~1%) including encephalopathy, meningitis, pneumonitis, myocarditis, esophagitis, colitis, hepatitis, and nephritis, in addition to myositis and arthritis (Samaik, A.A. et al, 2011; Wolchok, J.D. et al. 2010).

Anti-PD-1 anti CTLA-4 combined treatment show grade 3-4 adverse event in 59% of melanoma patients, with higher incidence of gastrointestinal event (Wolchok JD, 2017).

Failure of ICI therapy can result from inadequate T-cell priming. Full T-cell activation depends on the interaction of T-cell receptor (TCR) with peptide bound to major histocompatibility complex, but also on costimulatory signals provided by non-T accessory cells (Mueller DL et al, 1989; Esensten JH, 2016). Costimulatory receptors belong to either the immunoglobulin superfamily (e.g., CD28, ICOS, and CD226) or the tumour necrosis factor receptor superfamily (TNFRSF), including CD27, OX40 (also referred to as CD-134), 4-1BB, glucocorticoid-induced TNF receptor related protein, death receptor 3, and CD30 (Dougall WC et al., 2017; Wikenheiser DJ et al., 2016; Ward-Kavanagh LK et al., 2016). Costimulatory signals boost the magnitude of the T-cell response and the generation of effector and memory T cells, and may promote cellular immune responses against tumors.

OX40 agonist antibody promotes T-cell activation and antitumor immunity in several clinical studies and synergizes with PD-L1 blockade to enhance the proliferation and function of exhausted CD8 T cells. The most common adverse effect of OX40 monotherapy are lymphopenia, fatigue, rash, and flu-like symptoms (grade 1-2) (Weinberg AD et al., 2000; Piconese S et al., 2008; Bulliard Y et al., 2014, Curti BD et al., 2013).

However, the combination of anti-OX40 with PD-1/PD-L1 checkpoint blockade produce a higher frequency of immune related adverse event (irAEs) than the respective individual treatments (Montler R et al., 2016).

Therefore, there is still a need for a therapy that increases the response to immunotherapeutic agents and reduces their side effects, adverse events and toxicity.

### SUMMARY OF THE INVENTION

In the present invention, the authors have identified that the combination of a specific caloric intake regime with at least one immunotherapeutic agent is effective in the treatment of cancer. Said specific caloric intake regime is based on a reduced daily caloric intake compared to a regular daily caloric intake, in particular it involves a specific daily caloric intake and a specific macronutrient intake as defined below.

The invention is based on the surprising finding that a reduced caloric intake or a fasting mimicking diet (FMD) enhances the therapeutic activity of immunotherapeutic agent in the treatment of cancer. Surprisingly and unexpectedly, the combination of the invention is effective on cancers characterized by resistance or partial response to the treatment with at least one immunotherapeutic agent.

Therefore the invention provides at least one reduced caloric intake cycle and at least one PD-L1 inhibitor for use in the treatment of cancer wherein said at least one reduced caloric intake cycle comprises a first part with a regular caloric intake reduced by 30% to 70% and a second part with a regular caloric intake reduced by 40 to 97% and wherein said at least one PD-L1 inhibitor is an anti-PDL1 antibody.

Preferably said first part and/or said second part lasts for a period of 24 to 190 hours, preferably said first part and/or or said second part lasts for a period of 24 to 120 hours, preferably said first part and/or or said second part lasts for approximately 120 hours.

Preferably the at least one reduced caloric intake cycle is repeated from 1 to 30 times after respective periods of from 5 to 60 days.

In other words, each cycle may be separated by 5 to 60 days.

Preferably the PD-L1 inhibitor is selected from the group consisting of: atezolizumab, durvalumab, avelumab, APL-502, bintrafusp alfa, CS-1001, KN-035, SHR-1316, BGBA-333, CX-072, GEN-1046, GS-4224, KD-005, KLA-167, KN-046, STIA-1014, ADG-104, AK-106, BCD-135, cosibelimab, FAZ-053, FPT-155, FS-118, HLX-20, IBI-318, INBRX-105, JS-003, lodapolimab, LP-002, LY-3434172, MCLA-145, MSB-2311, SHR-1701, SL-279252, STIA-1015 or a combination thereof.

Preferably the PD-L1 inhibitor is atezolizumab.

Preferably the at least one reduced caloric intake cycle and at least one immunotherapeutic agent comprise administering a combination of:
- PD-L1 inhibitor and CTLA-4 inhibitor; or
- PD-L1 inhibitor and OX40 activator.

Preferably the at least one reduced caloric intake cycle and at least one immunotherapeutic agent for use as defined above, comprise administering a further therapeutic intervention. Preferably said further therapeutic intervention is selected from the group consisting of: surgery, radiotherapy and at least one further therapeutic agent.

Preferably said further therapeutic agent is a further immune checkpoint inhibitor, an immune response stimulator, a targeted anticancer agent, a DNA Damage Response inhibitor and/or a chemotherapeutic agent.

Preferably said further immune checkpoint inhibitor is selected from the group consisting of: PD1 inhibitors, PDL1 inhibitors, CTLA-4 inhibitors, TIGIT inhibitors, ICOS inhibitors, TIM3 inhibitors, IDO1 inhibitors; said immune response stimulator is selected from the group consisting of: OX40 activators, GITR modulators, 4-1BB agonists; said targeted anticancer agent is selected from the group consisting of: PI3K inhibitors, HDAC inhibitors, EGFR inhibitors, BRAF inhibitors, MAPK inhibitors, CDK inhibitors, ER stress activators; said DNA Damage Response inhibitor is selected from the group consisting of: PARP inhibitors, CHK1 inhibitors, ATR inhibitors, Wee1 inhibitors; said chemotherapeutic agent is selected from the group consisting of: Alkylating agents, Antimetabolites, Anti-microtubule agents, Topoisomerase inhibitors, Cytotoxic antibiotics.

Preferably said cancer is characterized by resistance or partial response to the treatment with at least one immunotherapeutic agent, preferably said cancer is resistant or has a partial response to a least one PD-1 inhibitor, PD-L1 inhibitor, CTLA-4 inhibitor or OX-40 activator. Preferably said cancer is resistant or has a partial response to pembrolizumab and/or atezolizumab.

The cancer may be resistant to other agents such as commonly used chemotherapy.

Preferably said cancer is a solid or hematopoietic cancer, preferably the cancer is selected from the group consisting of: breast cancer, melanoma, lymphoma, lung cancer, non-small cell lung cancer (NSCLC), head and neck cancer, gastroesophageal cancer, bladder cancer and urothelial cancer, hepatocellular carcinoma and renal cell carcinoma.

The present invention provides a treatment of cancer comprising
- administering at least one reduced caloric intake cycle; and
- administering at least one immunotherapeutic agent,
wherein said at least one reduced caloric intake cycle comprises a first part with a regular caloric intake reduced by 30% to 70% and a second part with a regular caloric intake reduced by 40 to 97% and wherein the immunotherapeutic agent is an anti-PDL1 antibody.

Preferably the cancer is characterized by resistance or partial response to the treatment with at least one immunotherapeutic agent.

Preferably said reduced caloric intake comprises a reduced protein intake and/or a reduced simple carbohydrate intake and/or an increased complex carbohydrate intake and/or an increased unsaturated fat intake.

Preferably said increased complex carbohydrate intake is approximately from 40% to 50% of total caloric intake, preferably said reduced protein intake is approximately from 9 to 11% of total caloric intake, preferably said increased complex carbohydrate intake is approximately from 43 to 47% of total caloric intake, preferably said increased unsaturated fat intake is approximately from 44 to 46% of total caloric intake.

In a preferred embodiment the reduced caloric intake is carried out by administering a specific regimen. Said regimen consists of a 4 days regimen. It provides approximately 1.100 kilocalories for a first day and less than 300 kilocalories per day for a second to fourth day of the diet (Table 1). It includes less than 30 grams of sugar on the first day; less than 5 grams of sugar on the second to fourth days; less than 30 grams of proteins on the first day; less than 5 grams of proteins on days the second to fourth days; less than 15 grams of saturated fats on the first day (Tables 2 and 3).

**Table 1. Exemplary of Fasting mimicking diet (FMD) developed to induce a fasting-like response while maximizing nourishment.**

| | **Day 1** | **Day 2** | **Day 3** | **Day 4** |
|---|---|---|---|---|
| **Total Calorie** | 1.112 | 240 | 238 | 153 |

**Table 2. The macronutrient content for each day of the 4 day FMD. *Avarage values.**

| | **Day 1** | ***Day 2, 3, 4** |
|---|---|---|
| **Total Calorie** | 1.112 | ~ 210 |
| Fats | ~ 61 % | ~ 8% |
| Carbohydrates | ~ 31 % | ~ 80 % |
| *(of which sugars)* | (~ 9 %) | ~ 6 % |
| Proteins | ~ 9 % | ~ 5 % |

**Table 3. The micronutrient content for each day of the 4 day FMD regimen based on an average 180-2001bs person.**

| | **Unit** | **Day 1** | **Day 2,3,4*** |
|---|---|---|---|
| **Total Fat** | (g) | 75 | 2 |
| **% DV** | | 96 | 2,3 |
| **Protein** | (g) | 25 | 2,6 |
| **% DV** | | 51 | 5 |
| **Total** | (g) | 103 | 45,6 |
| **Carbohydrate % DV** | | 37 | 13,3 |
| **Sugars** | (g) | 26,30 | 3,1 |
| **Dietary Fiber** | (g) | 32 | 5,7 |
| **% DV** | | 113 | 19,6 |
| **VIT A** | (IU) | 6.442 | 1.489 |
| **% DV** | | 716 | 157 |
| **VIT C** | (mg) | 9 | 15,6 |
| **% DV** | | 101 | 14.35 |
| **Calcium** | (mg) | 445 | 52,6 |
| **% DV** | | 34 | 5 |
| **Iron** | (mg) | 16 | 1,6 |
| **% DV** | | 91 | 8 |
| **Sodium** | (mg) | 1.970 | 1.148 |
| **% DV** | | 82 | 49,6 |
| **Potassium** | (mg) | 1.088 | 324,3 |
| **% DV** | | 23 | 6,6 |

| | | | |
|---|---|---|---|
| % DV indicates the percent of daily value based on a 2.000 calorie diet updated to 2020 regulations. *Avarage values | | | |

Embodiments and experiments illustrating the principles of the invention will be discussed with reference to the following figures.

### Brief description of the figures

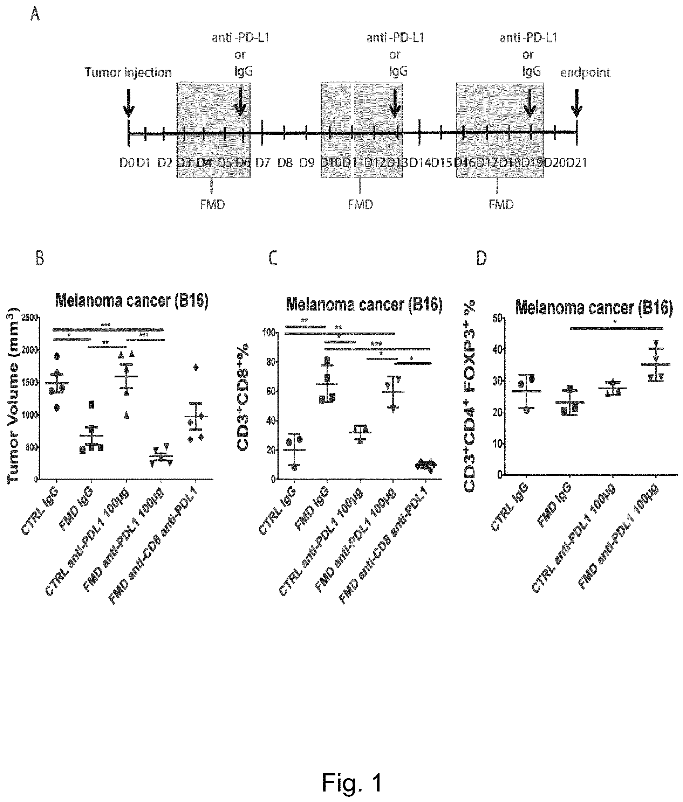

**Figure 1****.** Fasting mimicking diet (FMD) and anti-PD-L1 synergize to mediate B16F10 melanoma tumour growth inhibition by increasing CD3⁺CD8⁺ Tumour infiltrating lymphocytes (TIL). Treatment schedule (A), mean tumour size (B), CD3⁺CD8⁺ TIL (C), Tregs (CD3⁺CD4⁺FoxP3⁺) (D) for C57BL/6 mice bearing B16F10 melanoma cells fed with standard diet (CTRL) or FMD and treated with anti-PD-L1 (100µg/mouse), IgG (100µg/mouse), anti-CD8 (200 µg/mouse). Data shown are pooled from 3 independent experiments with 4 mice per group. One-way ANOVA was used to evaluate statistical significance (*, P < 0.05; **, P < 0.01; ***, P < 0.001; ****, P < 0.0001; NS, P < 0.1).

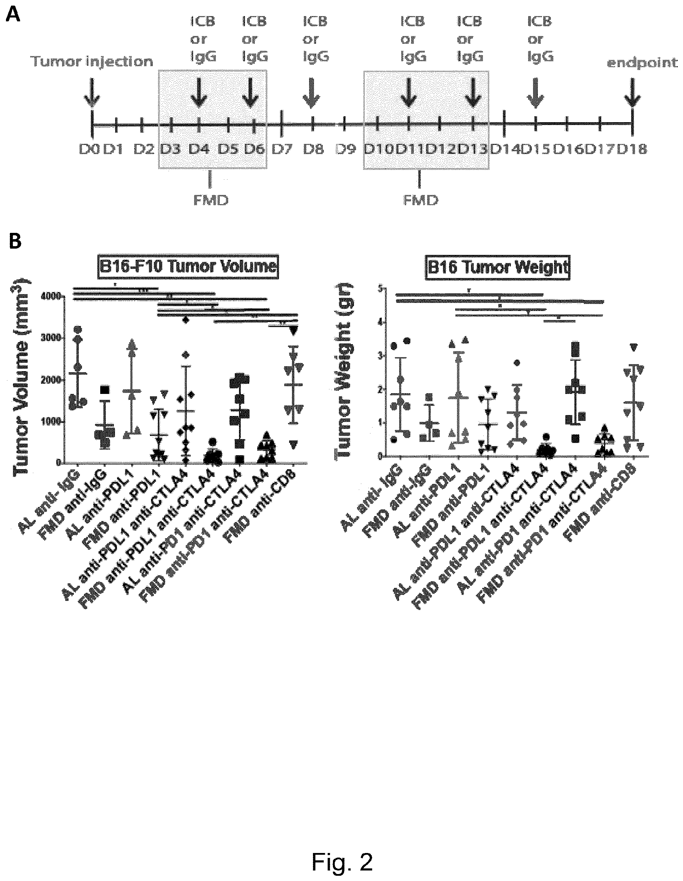

**Figure 2****.** Fasting mimicking diet in combination with Immune checkpoint blockade (ICB), anti-PD-L1/CTLA4 or anti-PD1/CTLA4, is effective in halting B16F10 melanoma tumour progression. Treatment schedule (A), mean tumour size (B), mean tumour weight (C) for C57BL/6 mice bearing B16F10 melanoma cells fed with standard diet (AL) or FMD and treated with immune checkpoint blockade (anti-PD-L1 100µg/mouse, anti-PD1 100µg/mouse and anti-CTLA-4 100µg/mouse). Data shown are pooled from 3 independent experiments with 4 mice per group. One-way ANOVA was used to evaluate statistical significance (*, P < 0.05; **, P < 0.01; ***, P < 0.001; ****, P < 0.0001; NS, P < 0.1).

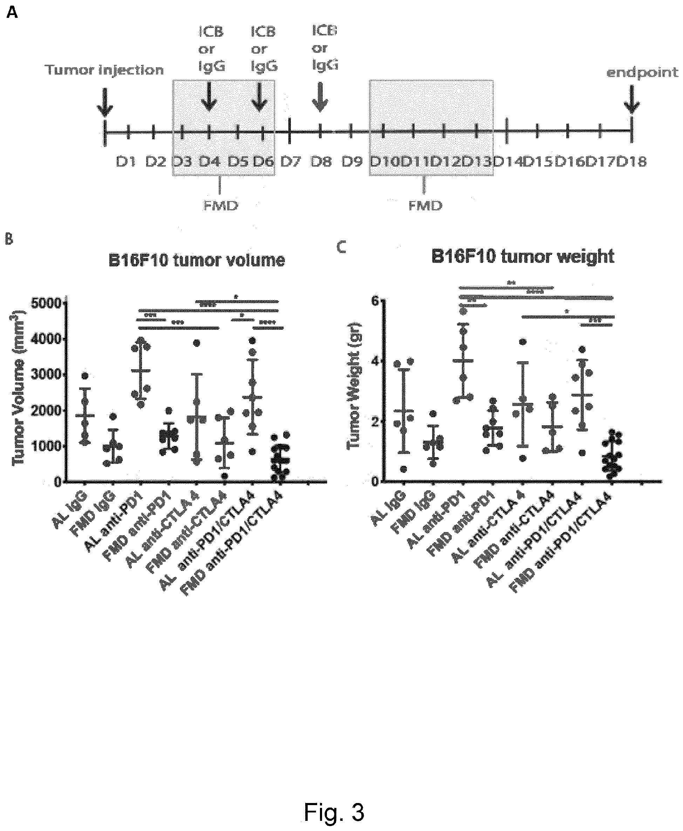

**Figure 3****.** FMD in combination with anti-PD1/CTLA4 or anti-PD-L1/CTLA4halts B16 tumour growth. Treatment schedule (A), mean tumour size (B), mean tumour weight (C) for C57BL/6 mice bearing B16F10 melanoma cells fed with standard diet (AL) or FMD and treated with immune checkpoint blockade( ICB) (anti-PD1 250µg/mouse, and anti-CTLA-4 200µg/mouse). Data shown are pooled from 3 independent experiments with 4 mice per group. One-way ANOVA was used to evaluate statistical significance (*, P < 0.05; **, P < 0.01; ***, P < 0.001; ****, P < 0.0001; NS, P < 0.1).

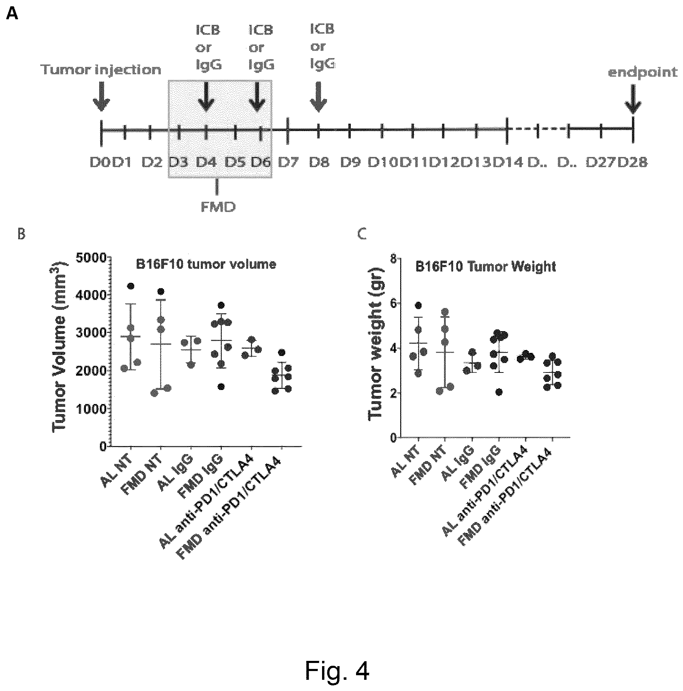

**Figure 4****.** One cycle of FMD enhances the antitumoral response of anti-PD1/CTLA4 treatment against B16 melanoma tumour. Treatment schedule (A), mean tumour size (B), mean tumour weight (C) for C57BL/6 mice bearing B16F10 melanoma cells fed with standard diet ad libitum (AL) or FMD and treated with immune checkpoint blockade ICB (anti-PD1 250µg/mouse and anti-CTLA-4 200µg/mouse).

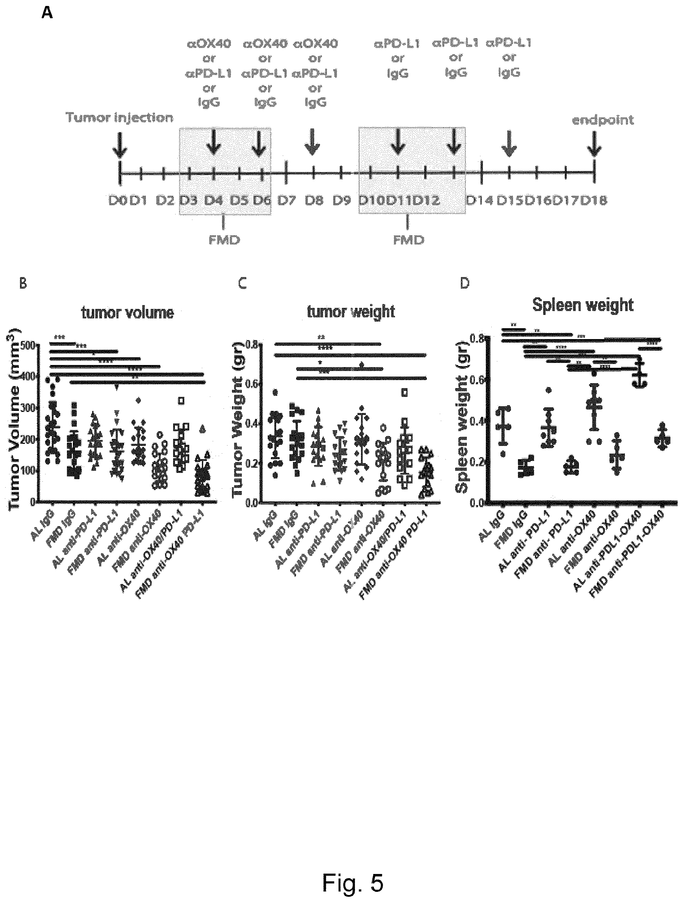

**Figure 5****.** FMD boosts the anti-tumoral response of anti-PD-L1/OX40 therapy against 4T1 breast tumour and prevents spleen enlargement. Treatment schedule (A), mean tumour size (B), mean tumour weight (C) and mean spleen weight (D) for BALB/c mice bearing 4T1 breast tumour fed with standard diet ad libitum (AL) or FMD and treated with immune checkpoint blockade (anti-PD-L1 100µg/mouse and anti-OX40 100µg/mouse). Data shown are pooled from 3 independent experiments with 4 mice per group. One-way ANOVA was used to evaluate statistical significance (*, P < 0.05; **, P < 0.01; ***, P < 0.001; ****, P < 0.0001; NS, P < 0.1).

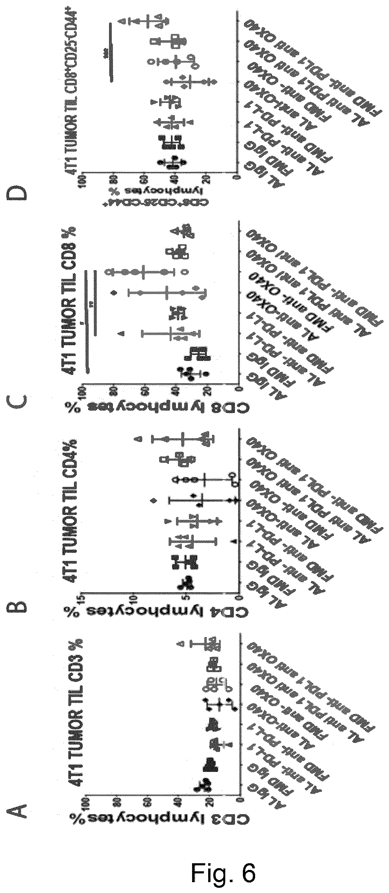

**Figure 6****.** FMD increases CD8⁺ and CD8⁺CD25⁻CD44⁺ tumour infiltrating lymphocytes (TIL, activated by tumour antigens). CD3⁺ Tumour infiltrating lymphocytes (A), CD4⁺ TIL (B), CD8+ TIL (C) such cells include activated CD8⁺CD25⁻CD44⁺ cells, activated CD8+CD25-CD44+ TIL (D) for BALB/c mice bearing 4T1 breast tumour fed with standard diet ad libitum (AL) or FMD and treated with anti-PD-L1 (100µg/mouse) and/or OX40 (100µg/mouse). Data shown are pooled from 3 independent experiments with 4 mice per group. One-way ANOVA was used to evaluate statistical significance (*, P < 0.05; **, P < 0.01).

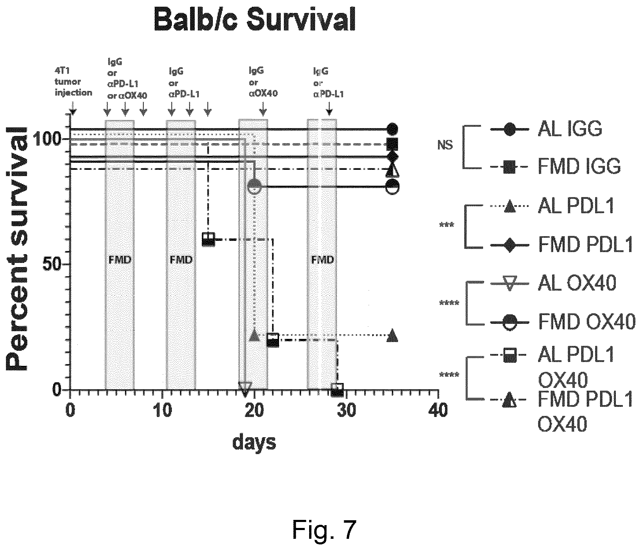

**Figure 7****.** FMD protects mice from anti-PDL1 and OX40 immune cytotoxicity. Beneficial effect of FMD in preventing anti-PD-L1 and/or anti-OX40 adverse effect on the survival of 4T1 allograft mice. Mice fed with standard diets ad libitum (AL) die upon the 4^{th} injection of anti-PD-L1 or anti-OX40, whereas mice fed with FMD (4 cycles) are much more tolerant and do not show any distress or adverse effect. Data shown are pooled from 3 independent experiments with 4 mice per group. One-way ANOVA was used to evaluate statistical significance (*, P < 0.05; **, P < 0.01; ***, P < 0.001; ****, P < 0.0001; NS, P < 0.1).

### DETAILED DESCRIPTION OF THE INVENTION

The present invention provides a reduced caloric intake and at least one immunotherapeutic agent for the treatment of cancer. **INSERT PAGE 9A**

Without being bound to any theory, the present data show that at least one reduced caloric intake cycle enhances the therapeutic activity of immunotherapeutic agent and/or increases CD8+ T cell population and/or reduces the toxicity of immunotherapeutic agent and/or prevents splenomegaly induced by immunotherapeutic agent.

The reduction is compared to a regular caloric intake per day. Regular caloric intake per day is between 1200 Kcal and 3000 Kcal. Preferably regular caloric intake per day (the range is based on age, sex and physical activity) is:
Age 4-8 years: 1200-2000 Kcal Age 9-13 years: 1800-2600 Kcal Age 19-30 years: 1800-3000 Kcal Age 31-50 years: 1800-2600 Kcal
+51 years: 1600-2600 Kcal.

In an embodiment, the reduced caloric intake for use according to the present invention lasts for a period of 24 to 190 hours, preferably said reduced caloric intake lasts for a period of 24 to 120 hours, preferably said reduced caloric intake lasts for approximately 120 hours.

In a preferred embodiment the period of reduced caloric intake is of 48 to 168 hours, preferably 120 hours.

Preferably the reduced caloric intake starts at least 24 hours before the immunotherapeutic agent is administered. Preferably the reduced caloric intake starts at least 48 hours before the immunotherapeutic agent is administered. Preferably the reduced caloric intake lasts at least 24 hours after the immunotherapeutic agent is administered, preferably it lasts at least 48, 72, 96, 120 hours after the immunotherapeutic agent is administered.

Preferably the reduced caloric intake is started one day before the immunotherapeutic agent is administered and continues for the following 2-4 days after immunotherapeutic agent administration (i.e. while the immunotherapeutic agent is most active). Preferably the reduced caloric intake consists of 4 days of low-calorie intake (50% of regular calorie intake on day 1, and 10% on days 2-4).

In the present invention, preferably, the reduced caloric intake is obtained by fasting or by means of dietetic food with reduced caloric and/or protein content but containing all necessary micronutrients to prevent malnutrition.

In the present invention, preferably, the reduced caloric intake is obtained by fasting mimicking diet (FMD). The term "fasting mimicking diet" (FMD) means a diet that mimics the effects of fasting typically by providing a subject with at most 50% of his normal caloric intake but with The invention is defined by the claims and any other aspects, configurations or embodiments set forth herein not falling within the scope of the claims are for information only.

some nutritional component, so that fasting is mimicked while a subject is not completely starved. Examples of useful fasting mimicking and enhancing in the context of the present invention are set forth in U.S. Pat. Appl. Nos. 14/273946 filed May 9, 2014; 14/497752 filed September 26, 2014; 12/910508 filed October 22, 2010; 13/982307 filed February 8, 2012; 14/060494 filed October 22, 2013; 14/178953 filed February 12, 2014; 14/320996 filed July 1, 2014; 14/671622 filed March 27, 2015. Additional examples of FMD diets are found in U.S. Pat. Appl. No. 15/148,251 and WIPO Pub. No. WO 2011/050302 and WIPO Pub. No. WO 2011/050302.

In the present invention the reduced caloric intake period is repeated one or more times after respective periods of 5-60 days, during which said mammal is given the agent while being subjected to a diet involving a regular caloric intake.

Preferably, the reduced caloric intake period is repeated from 1 to 3 times after respective period of 5-60 days.

Immunotherapy is the treatment of disease by activating or suppressing the immune system. Immunotherapies designed to elicit or amplify an immune response are classified as activation immunotherapies or immunostimulatory therapies, while immunotherapies that reduce or suppress are classified as suppression immunotherapies or immunosuppression therapies. Preferably, immunotherapy relates to anticancer immunostimulatory therapies harnessing pre-existing, ineffective, immune responses by targeting the immune checkpoint pathways. Preferably immunotherapeutic agents are: (1) drugs targeting the tumour immune evasion via blockade of negative regulatory signals (e.g., co-inhibitory checkpoints and tolerogenic enzymes) and (2) agents that directly stimulate immunogenic pathways (e.g., agonists of costimulatory receptors).

Additional immunostimulatory strategies include enhancers of antigen presentation (e.g., vaccines), the use of exogenous recombinant cytokines, oncolytic viruses, and cell therapies using native or modified antigen-competent immune cells.

The immunotherapeutic agents according to the present disclosure can be antibodies or antibody derivatives or fragments thereof. Among the antibody fragments are functional equivalents or homologues of antibodies including any polypeptide comprising an immuno- globulin binding domain or peptides mimicking this binding domain. Chimeric molecules comprising an immunoglobulin binding domain, or equivalents, fused to another polypeptide are therefore included. Preferably, the antibody derivative comprises at least parts of the Fab fragment, preferably together with at least parts of the F(ab')2 fragment and/or parts of the hinge region and/or the Fc part of a lambda or kappa antibody. Exemplary antibody molecules are intact immunoglobulin molecules and those portions of an immunoglobulin molecule that contains the para- tope, including those portions known as Fab, Fab', F(ab')2and F (v). Preferably, the antibody is an IgG, IgM or IgA antibody.

The antibody or antibody derivative used according to the invention can also be a glycosylated antibody, wherein the glycosylation can also mimic an epitope of a carbohydrate epitope of a tumour associated antigen (TAA).

The antibody or antibody derivative can be of human or animal origin, preferably of mammalian origin, for example of mouse, rat, goat origin. It can be produced by hybridoma technology according to methods well known from the art or by recombinant expression using appropriate expression systems. Depending on the host system used, the antibody or antibody derivative can show specific glycosylation patterns.

The immunotherapeutic agent according to the disclosure can be an anti-idiotypic antibody, i.e. an ab2 and/or an idiotypic antibody having specificity for a tumour associated antigen, i.e. an abl.

The immunotherapeutic agent according to the disclosure can also be a vaccine. This can be an antigenic structure, for example a TAA protein or polypeptide of a TAA which can either alone or together with a vaccine adjuvant induce an immune response against the antigen. The TAA antigen can be either isolated or recombinantly produced by known techniques.

The immunotherapeutic agent according to the present disclosure can also be a small molecule.

In the context of the present disclosure, a "derivative" or "analogue" of an immunotherapeutic agent includes a chemical modification made for the purpose of improving its properties, especially its pharmacokinetic, pharmacodynamic, chemical or physical properties. For example, a derivative may be a chemical modification made to the inhibitor for the purpose of increasing its binding affinity towards the receptor, increase its bioavailability or half-life.

In an embodiment, the immunotherapeutic agent for use according to the present disclosure is selected from the group comprising: PD-1 inhibitor, PD-L1 inhibitor, CTLA-4 inhibitor, OX40 activator, or derivative thereof, or a combination thereof.

Programmed death-1/ PD-1 (or CD279) is an immune checkpoint receptor and belongs to the B7-CD28 family of receptors. Upon binding to either of its two ligands, PD-L1 (known also as CD274 or B7-H1) and PD-L2 (known also as CD273, B7-DC or PDCD 1LG2), a co-inhibitory signal is delivered. PD-1 is a 55-kDa monomeric type I surface transmembrane glycoprotein. PD-1 is an immune checkpoint and guards against autoimmunity through two mechanisms. First, it promotes apoptosis (programmed cell death) of antigen-specific T-cells in lymph nodes. Second, it reduces apoptosis in regulatory T cells (anti-inflammatory, suppressive T cells). PD-1 inhibitors, a new class of drugs that block PD-1, activate the immune system to attack tumors and are used to treat certain types of cancer.

In a non-claimed aspect, the immunotherapeutic agent for use in the treatment of cancer is a PD-1 inhibitor.

In a non-claimed aspect, the immunotherapeutic agent for use in the treatment of cancer is a PD-1 inhibitor selected from the group consisting of: nivolumab, pembrolizumab, cemiplimab, camrelizumab, sintilimab, toripalimab, tislelizumab, AK-105, dostarlimab, HLX-10, prolgolimab, SCTI-10A, spartalizumab, AK-103, AK-104, APL-501, balstilimab, BAT-1306, BI-754091, cetrelimab, CS-1003, GLS-010, MGA-012, pidilizumab, sasanlimab, AMG-404, BCD-217, BH-2950, budigalimab, CC-90006, F-520, HAB-21, HX-009, IBI-318, JTX-4014, LY-3434172, LZM-009, MEDI-5752, MGD-013, MGD-019, ONO-4685, RO-7121661, RO-7247669, sulituzumab, Sym-021, XmAb-20717, XmAb-23104 or a derivative thereof, or a combination thereof.

In a non-claimed aspect, the immunotherapeutic agent for use in the treatment of cancer is pembrolizumab.

Programmed death-ligand 1 (PD-L1) also known as cluster of differentiation 274 (CD274) or B7 homolog 1 (B7-H1) is a protein that in humans is encoded by the CD274 gene.

The 40-kDa PD-L1 and the 25-kDa PD-L2 are both type I transmembrane proteins, containing extracellular IgV and IgC domains and a transmembrane domain. They lack an identifiable intracellular signalling domain. The two ligands share 37% identity with each other, but differ significantly in their affinity for PD-1 and their tissue specific expression.

PD-L1 expression/upregulation has been documented in various tumours, including melanoma, non-small cell lung cancer (NSCLC), breast cancer and squamous cell head and neck cancer.

Binding of PD-L1 to its receptor suppresses T cell migration, proliferation, and secretion of cytotoxic mediators, and restricts tumour cell killing. Inhibitors of PD-1 and PD-L1 disrupt PD-1 axis thereby reverses T cell suppression and enhances endogenous antitumor immunity to unleash long-term antitumor responses for patients with a wide range of cancers.

According to the invention, the immunotherapeutic agent for use in the treatment of cancer is a PD-1 inhibitor, namely an anti-PDL1 antibody.

In a preferred embodiment, the immunotherapeutic agent for use in the treatment of cancer is a PD-L1 inhibitor selected from the group consisting of: atezolizumab, durvalumab, avelumab, APL-502, bintrafusp alfa, CS-1001, KN-035, SHR-1316, BGBA-333, CX-072, GEN-1046, GS-4224, KD-005, KLA-167, KN-046, STIA-1014, ADG-104, AK-106, BCD-135, cosibelimab, FAZ-053, FPT-155, FS-118, HLX-20, IBI-318, INBRX-105, , JS-003, lodapolimab, LP-002, LY-3434172, MCLA-145, MSB-2311,SHR-1701, SL-279252, STIA-1015 or a combination thereof.

In a preferred embodiment, the immunotherapeutic agent for use in the treatment of cancer is atezolimumab.

CTLA4 or CTLA-4 (cytotoxic T-lymphocyte-associated protein 4), also known as CD152 (cluster of differentiation 152), 4 is a type I membrane protein that is expressed on activated T cells and monocytes, which mediates a local and temporary inhibition of the immune system. CTLA4 is constitutively expressed in regulatory T cells but only upregulated in conventional T cells after activation - a phenomenon which is particularly notable in cancers. It acts as an "off" switch when bound to CD80 or CD86 on the surface of antigen-presenting cells. CTLA-4 inhibitors can abolish the inhibition on the immune system, resulting in sustained immune-mediated antitumor activity.

In a non-claimed aspect, the immunotherapeutic agent for use in the treatment of cancer is a CTLA-4 inhibitor.

In a non-claimed aspect, the immunotherapeutic agent for use in the treatment of cancer is a CTLA-4 inhibitor is selected from the group consisting of: ipilimumab, tremelimumab, zalifrelimab, AK-104, BMS-986218, BMS-986249, KN-046, ADU-1604, AGEN-1181, ATOR-1015, BCD-145, BCD-217, FPT-155, HBM-4003, IBI-310, MEDI-5752, MGD-019, MK-1308, REGN-4659, RP-2, XmAb-20717, XmAb-22841, PSB-205, ALPN-202, APL-509, BPI-002, BT-001, CBT-103, CBT-107, CG-0161, HL-06, HLX-09, JS-007, KN-044, MV-049, ONC-392, PC-101, BJ-003, DB-002, IMT-400, JMW-3B3, TE-1254, AGEN-2041, FHTCT-4, HOR-010, PRS-010, SNCA-21 or a derivative thereof, or a combination thereof.

In a non-claimed aspect, the immunotherapeutic agent for use in the treatment of cancer is a CTLA-4 inhibitor is ipilimumab.

Tumour necrosis factor receptor superfamily, member 4 (TNFRSF4), also known as CD134 and OX40 receptor, is a member of the TNFR-superfamily of receptors which is not constitutively expressed on resting naive T cells, unlike CD28. OX40 is a secondary costimulatory immune checkpoint molecule, expressed after 24 to 72 hours following activation; its ligand, OX40L, is also not expressed on resting antigen presenting cells, but is following their activation. Expression of OX40 is dependent on full activation of the T cell; without CD28, expression of OX40 is delayed and of fourfold lower levels.

Agonistic antibodies to OX40 promote effector T-cell response and induce tumour regression. In a non-claimed aspect, the immunotherapeutic agent for use in the treatment of cancer is an OX40 activator.

In a non-claimed aspect, the immunotherapeutic agent for use in the treatment of cancer is an OX40 activator selected from the group consisting of: BMS-986178, GSK-3174998, INCAGN-1949, KHK-4083, ABBV-368, ATOR-1015, DNX-2440, IBI-101, SL-279252, INBRX-106, AP-201, APVO-603, DPV-002, FS-120, HLX-51, JNJ-6892, MSB-013, OrthomAb, ABM-193, HuOHX-10, INV-531, SCB-340, ENUM-004, GBR-8383, KAHR-104, MEDI-6469, ZL-1101, efizonerimod alfa, tavolimab, vonlerolizumab or a derivative thereof, or a combination thereof.

In a non-claimed aspect, the immunotherapeutic agent for use in the treatment of cancer is an OX40 activator is BMS-986178.

In one embodiment, the reduced caloric intake and at least one immunotherapeutic agent for use according to anyone of previous claims, comprises administering a combination of:
- PD-L1 inhibitor and CTLA-4 inhibitor; or
- PD-L1 inhibitor and OX40 activator.

In one embodiment, the reduced caloric intake and at least one immunotherapeutic agent for use according to the invention are combined with a further therapeutic intervention.

In a preferred embodiment, the reduced caloric intake and at least one immunotherapeutic agent for use according to the invention are combined with a further therapeutic intervention selected from the group consisting of: surgery, radiotherapy and at least one further therapeutic agent. Table 4 shows a list of drug classes that can be used in combination with the reduced caloric intake and the at least one immunotherapeutic agent for use according to the invention.

**Table 4. Drug classes that can be used in combination with the reduced caloric intake and the at least one immunotherapeutic agent.**

| **Drug Class** | **Drugs** | **Examples** |
|---|---|---|
| Immune checkpoint inhibitors | PD1 inhibitors | anti-PD1 monoclonal antibodies |
| | PDL1 inhibitors | anti-PDL1 monoclonal antibodies |
| | CTLA-4 inhibitors | anti-CTLA4 monoclonal antibodies |
| | TIGIT inhibitors | anti-TIGIT monoclonal antibodies |
| | ICOS inhibitors | anti-ICOS monoclonal antibodies |
| | TIM3 inhibitors | anti-TIM3 monoclonal antibodies |
| | IDO1 inhibitors | Epacadostat monoclonal antibodies |
| Immune response stimulators | OX40 activators | anti-OX40 monoclonal antibodies |
| | GITR modulators | anti-GITR monoclonal antibodies |
| | 4-1BB agonists | anti-4-1BB monoclonal antibodies |
| Targeted anticancer agent | PI3K inhibitors | Buparlisib |
| | HDAC inhibitors | Entinostat |
| | EGFR inhibitors | Cetuximab |
| | BRAF inhibitors | Vemurafenib |
| | MAPK inhibitors | Dabrafenib |
| | CDK inhibitors | Palbociclib |
| | ER stress activators | Honokiol |
| DNA Damage Response Inhibitors | PARP inhibitors | Olaparib |
| | CHK1 inhibitors | LY2603618 |
| | ATR inhibitors | AZD6738 |
| | Wee1 inhibitors | AZD1775 |
| Chemotherapeutic Drugs | Alkylating agents | Cyclophosphamide |
| | Antimetabolites | 5-fluorouracil |
| | Anti-microtubule agents | Paclitaxel |
| | Topoisomerase inhibitors | Camptothecin |
| | Cytotoxic antibiotics | Bleomycin |

In a preferred embodiment, the reduced caloric intake and at least one immunotherapeutic agent for use according to the invention are combined with a further therapeutic agent is a further immune checkpoint inhibitor, an immune response stimulator, a targeted anticancer agent, a DNA Damage Response inhibitor and/or a chemotherapeutic agent.

In a preferred embodiment, the reduced caloric intake and at least one immunotherapeutic agent for use according to the invention are combined a further immune checkpoint inhibitor selected from the group consisting of: PD1 inhibitors, PDL1 inhibitors, CTLA-4 inhibitors, TIGIT inhibitors, ICOS inhibitors, TIM3 inhibitors, IDO1 inhibitors, and/or an immune response stimulator selected from the group consisting of: OX40 activators, GITR modulators, 4-1BB agonists, and/or a targeted anticancer agent selected from the group consisting of: PI3K inhibitors, HDAC inhibitors, EGFR inhibitors, BRAF inhibitors, MAPK inhibitors, CDK inhibitors, ER stress activators, and/or a DNA Damage Response inhibitor selected from the group consisting of: PARP inhibitors, CHK1 inhibitors, ATR inhibitors, Wee1 inhibitors, and/or a chemotherapeutic agent selected from the group consisting of: Alkylating agents, Antimetabolites, Anti-microtubule agents, Topoisomerase inhibitors, Cytotoxic antibiotics. Non limitative examples of immune checkpoint inhibitors include: nivolumab, pembrolizumab, cemiplimab, camrelizumab, sintilimab, toripalimab, tislelizumab, atezolizumab, durvalumab, avelumab, APL-502, bintrafusp alfa, ipilimumab, tremelimumab, zalifrelimab, AK-104, BMS-986207, tiragolumab, AB-154, AMG-570, ELN-21, ELN-12, darvadstrocel, indoximod, epacadostat.

Non limitative examples of immune response stimulators include: BMS-986178, GSK-3174998, INCAGN-1949, KHK-4083, ABBV-368, ATOR-1015, Utomilumab.

Non limitative examples of targeted anticancer agents include: alpelisib, copanlisib, rigosertib, dactolisib, duvelisib, entinostat, romidepsin, sodium phenylbutyrate, belinostat, panobinostat, osimertinib, cetuximab, panitumumab, erlotinib, vemurafenib, sorafenib, regorafenib, dabrafenib, miltefosine, brimapitide, acumapimod, palbociclib, abemaciclib, ribociclib, alvocidib, honokiol.

Non limitative examples of DNA Damage Response inhibitors include: olaparib, rucaparib camsylate, talazoparib, niraparib, LY2603618, AZD7762, SRA-737, CBP-501, ESP-01, prexasertib, AZD6738, berzosertib, M-4344, BAY-1895344, AZD1775, adavosertib, ZNC-3. Non limitative examples of chemotherapeutic agents include: Cisplatin, Carboplatin, Dicycloplatin, Oxaliplatin, Picoplatin, Satraplatin, Cyclophosphamide, Chlormethine, Uramustine, Melphalan, Chlorambucil, Ifosfamide, Bendamustine, Carmustine, Lomustine, Streptozocin, Busulfan, Procarbazine, Altretamine, Dacarbazine, Temozolomide, Mitozolomide.

Preferably, the reduced caloric intake and the at least one immunotherapeutic agent according to the present invention can be used in the treatment of a cancer characterized by resistance or partial response to the treatment with at least one immunotherapeutic agent.

Preferably, the reduced caloric intake and the at least one immunotherapeutic agent according to the present invention can be used in the treatment of a cancer characterized by a reduction in tumour mass or volume comprised between 0% and 90% after a treatment with at least one immunotherapeutic agent.

Preferably, the reduced caloric intake and the at least one immunotherapeutic agent according to the present invention can be used in the treatment of a cancer characterized by a reduction in tumour mass or volume comprised between 0% and 90% after a treatment with at least one immunotherapeutic agent according to claim 1.

Preferably, the reduced caloric intake and the at least one immunotherapeutic agent according to the present invention can be used in the treatment of a cancer selected from: breast cancer, melanoma, lymphoma, lung cancer, non-small cell lung cancer (NSCLC), head and neck cancer, gastroesophageal cancer, bladder cancer and urothelial cancer, hepatocellular carcinoma and renal cell carcinoma.

More preferably, the reduced caloric intake and the at least one immunotherapeutic agent according to the present invention can be used in the treatment of breast cancer or melanoma. The present invention further provides an in vitro method of treating a cancer cell with at least one agent as defined in above, comprising:
- cultivating the cancer cell in a medium with reduced serum or glucose concentration; and
- treating the cancer cell with the immunotherapeutic agent.
wherein the serum concentration in the medium is less than 10% and the glucose concentration in less than 1 g/1, preferably the serum concentration is less than 5% , still preferably the serum concentration is 1% or less than 1% . Preferably the glucose concentration is less than 0.8 g/liter, preferably less than 0.6 g/liter, still preferably 0.5 g/liter, preferably less than 0.5 g/liter. Preferably the serum concentration in the medium is reduced by 10-90% or the glucose concentration in the medium is reduced by 20-90%, the reduction is in respect of normal or control concentrations (i.e. 10 % of serum and 1 g/liter of glucose).

The present invention also provides a treatment of cancer comprising
- administering a reduced caloric intake; and
- administering at least one immunotherapeutic agent.
wherein cancer is characterized by resistance or partial response to the treatment with at least one immunotherapeutic agent, wherein the immunotherapeutic agent is a PD-L1 inhibitor, namely an anti-PDL1 antibody.

Wherein the reduced caloric intake lasts for a period of 24 hours to 190 hours and wherein said reduced caloric intake is a daily caloric intake reduced by 10 to 100%.

In the present invention a preferred reduced caloric intake is as follows:
Day 1: 54% caloric intake, about 1,090 kcal (10% protein, 56% fat, 34% carbohydrate)
Days 2-7: 20-34% caloric intake, about 426-725 kcal (5.3-9% protein, 26-44% fat, 27.6-47% carbohydrate).

The at least one immunotherapeutic agent for use according to the present invention can be incorporated into pharmaceutical compositions. Such compositions typically include the at least immunotherapeutic agent and at least one pharmaceutically acceptable carrier. As used herein the wording "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral, inhalation, transdermal (topical), transmucosal, and rectal administration.

In certain preferred embodiments, the pharmaceutical composition comprising at least one immunotherapeutic agent for use according to the present invention, used for parenteral, intradermal, or subcutaneous application, may further comprise one or more pharmaceutically acceptable carriers, exemplified by, but not limited to, lipid particles, lipid vesicles, liposomes, niosomes, sphingosomes, polymeric nanocarriers, nanoparticles, microparticles, nanocapsules, and nanospheres.

The pharmaceutical compositions of the present invention are preferably in the form of a single unit dosage form that contains an amount of the therapeutic agent that is effective to treat and/or prevent a cancer of the type described herein and at least one pharmaceutically acceptable excipient.

Suitable pharmaceutically acceptable excipients are those commonly known to the person skilled in the art for the preparation of compositions for parenteral, intradermal, subcutaneous, oral, transdermal, topical, transmucosal, and rectal administration.

By way of non-limiting example, said acceptable carriers can consists of binders, diluents, lubricants, glidants, disintegrants, solubilizing (wetting) agents, stabilizers, colorants, anti-caking agents, emulsifiers, thickeners and gelling agents, coating agents, humectants, sequestrants, and sweeteners.

The amount of the at least one immunotherapeutic agent in the pharmaceutical composition of the present invention will depend upon the nature and severity of the condition being treated, and on the nature of prior treatments which the patient has undergone. For a human patient, the attending physician will decide the dose of compound of the present invention with which to treat each individual patient. Initially, the attending physician can administer low doses and observe the patient's response. Larger doses may be administered until the optimal therapeutic effect is obtained for the patient, and at that point the dosage is not increased further. The duration of therapy using the pharmaceutical composition of the present invention will vary, depending on the severity of the disease being treated and the condition and potential idiosyncratic response of each individual patient.

In an embodiment, the invention provides a reduced caloric intake and at least one immunotherapeutic agent for use in the treatment of cancer, wherein the cancer is characterized by resistance or partial response to immunotherapy.

### Definitions

According to the present invention, preventing a disease refers to inhibiting completely, or in part, the development or progression of a disease, for example in a person who is known to have a predisposition to a disease. An example of a person with a known predisposition is someone with a history of cancer in the family, or who has been exposed to factors that predispose the subject to the development of a tumour.

Treating a disease refers to a therapeutic intervention that inhibits, or suppressed the growth of a tumour, eliminates a tumour, ameliorates at least one sign or symptom of a disease or pathological condition, or interferes with a pathophysiological process, after the disease or pathological condition has begun to develop.

Therapeutically effective dose is used in the context of the present invention to characterize an amount of the drug, which leads to complete or partial remission of the neoplastic disease. For instance, any statistically significant reduction in the mass or volume of the tumour or in the number of cancer cells indicates therapeutic efficacy in the context of the present invention. Pharmaceutically acceptable is used in the context of the present invention to refer to those compounds, materials, compositions, and/or dosage forms that are, within the scope of sound medical judgment, suitable for use in contact with the tissues of human beings and animals without excessive toxicity, irritation, allergic response, and/or other problem or complication, commensurate with a reasonable benefit/risk ratio.

Resistance is used in the context of the present invention to refer to no variation in tumour mass or volume after a treatment with at least one immunotherapeutic agent.

Partial response is used in the context of the present invention to refer to a decrease in the size of a tumour, or in the extent of cancer in the body, in response to treatment with at least one immunotherapeutic agent. Preferably, partial response is used in the context of the present invention to refer to a reduction in tumour mass or volume comprised between 0% and 90% after a treatment with at least one immunotherapeutic agent. Partial response can also be called partial remission.

Immunotherapy is used in the context of the present invention to refer to the prevention or treatment of disease with agents (i.e. immunotherapeutic agents) that stimulate the immune response.

According to the present invention, simultaneous administration may, e.g., take place in the form of one fixed combination with two or more active ingredients, or by simultaneously administering two or more active ingredients that are formulated independently. Sequential use (administration) preferably means administration of one (or more) components of a combination at one time point, other components at a different time point, that is, in a chronically staggered manner, preferably such that the combination shows more efficiency than the single compounds administered independently (especially showing synergism). Separate use (administration) preferably means administration of the components of the combination independently of each other at different time points.

Also combinations of two or more of sequential, separate and simultaneous administration are possible, preferably such that the combination component-drugs show a joint therapeutic effect that exceeds the effect found when the combination component-drugs are used independently at time intervals so large that no mutual effect on their therapeutic efficiency can be found, a synergistic effect being especially preferred.

### MATERIALS AND METHODS

### (1) Tumour cell lines.

The B16-F10 (ATCC^{®} CRL6475^{™}) and 4T1 (ATCC^{®} CRL-2539^{™}) tumour cell was obtained from ATCC. B 16F 10 are murine skin cancer cells used as model for human melanoma cancer. This cell line was derived from pulmonary melanoma nodule of the B 16-F0 parent cell line injected into C57BL/6 mouse tail vein. B16F10 cells are highly metastatic and will form tumors and metastases post implantation into syngenic C57BL/6 mice.

4T1 are murine breast cancer cells line derived from the mammary gland tissue of a mouse BALB/c and used as model for human triple negative breast cancer. 4T1 cells form tumors and spontaneous metastases post implantation into syngenic BALB/c mice.

B16 and 4T1 are also used as a pre-clinical model to study immunotherapy and both cells lines are resistant to immune checkpoint blockade therapy (anti-PD1, anti-PD-L1 and anti-CTLA4).Cells were grown in RPMI1640 or DMEM supplemented with 10% fetal bovine serum (FBS), 2 mM l-glutamine, penicillin (100 U ml-1) and streptomycin (100 µg ml-1) at 37 °C with 5% CO2 and maintained at a confluence of 70-80%.

### (2) Tumour implantation, Immune checkpoint blockade (ICB) treatment and tumour volume Measurement.

C57BL/6J and BALB/c female mice, 6-8 weeks old, were purchased from Charles River and housed under pathogen-free conditions in Cogentech animal facility and with food and water ad libitum. All procedures were carried out in accordance with approved Institutional Animal Care and Use Committee (OPBA) animal protocols at IFOM-the FIRC Institute of Molecular Oncology.

In the vivo experiments, tumours were implanted in C57BL/6J mice by injecting subcutaneously (s.c.) 2x10⁵ B16 tumour cells per mouse into the right flank at day 0. When tumours were palpable, mice from the appropriate groups (5 mice per group) were treated intraperitoneally (i.p.) with anti-PD-1 antibody, anti-CTLA4 antibody and anti-PD-L1 antibody at different dose and timepoint according to designed experimental conditions.

In the experiment shown in figure 1, IgG and anti-PD-L1 was administered 100µg/mouse once a week for 3 weeks. The mice belonging to FMD group underwent 3 cycles of FMD, once per week.

In the experiment of figure 2, IgG, anti-PD1, anti-PD-L1 and anti-CTLA4 were administered at a dose of 100µg/mouse three times a week for 2 weeks. Anti-CD8 antibody was administered at a dose of 200µg/mouse every 3 days. Mice belonging to FMD group underwent 2 cycles of FMD, once per week.

In the experiment of figure 3, mice were treated intraperitoneally (i.p.) with anti-PD-1 antibody (at the dose of 250 µg per mouse), anti-CTLA4 antibody (at dose of 200 µg per mouse the first injection and 100 µg per mouse the second and third injection) and IgG 450 µg per mouse. Mice belonging to FMD group underwent 2 cycles of FMD , once per week.

In the experiment of figure 4, mice were treated intraperitoneally (i.p.) with anti-PD-1 antibody (at the dose of 250 µg per mouse), anti-CTLA4 antibody (at dose of 200 µg per mouse the first injection and 100 µg per mouse the second and third injection) and IgG 450 µg per mouse. Mice belonging to FMD group underwent 1 cycle of FMD.

For triple negative breast cancer (TNBC) model, 3 x 10⁴ 4T1 cells were injected orthotopically into mouse mammary fat pad of 6-8-week-old BALB/c mice. When the tumours were palpable, mice were treated with IgG (100 µg per mouse) (anti-OX40 antibody 100 µg per mouse) and/or anti-PD-L1 antibody (100 µg per mouse) every other day for three times. Anti-OX40 was administered the first week while anti-PD-L1 was administered the second week to the group belonging to the combine treatment. Mice belonging to FMD group underwent 2 cycles of FMD, once per week (Figures 5 and 6).

As reported in the experimental scheme of figure 7, 3 days after the injection of 4T1 cells into the mammary fat pad, the mice undergo 1 cycle of FMD (4 days) every week for 4 weeks and 3 treatments with IgG, anti-PD -L1 and/or anti-OX40 3 times a week on alternate days for 1 week. The mice belonging to anti-PD-L1/OX40 group are treated the first week 3 times with anti-OX40, the second week 3 times with anti-PD-L1, while the mice of the anti-PD-L1 and OX40 group do not receive any treatment the second week. Starting from the third week, the mice are treated with IgG, anti-PD-L1 or anti-OX40 1 time per week on the last day of FMD. Survival of mice over time was monitored.

Tumours were measured every 3-4 days using a digital caliper; tumour volume was calculated using the formula V = (L × W x H )/2, where V is tumour volume, L is the length of the tumour (longer diameter), W is the width of the tumour (shorter diameter) and H is the height (diameter of tumour perpendicular to length and width). Mice were monitored for tumour growth and survival. Mice were killed when tumour volume reached 1.5 cm³.

The tumor masses, collected at experimental endpoint, were weighed on an analytical balance in order to compare the volume and mass.

### (3) Flow cytometry analysis of tumour-infiltrating lymphocytes and apoptosis.

At experimental endpoint, the mice were sacrificed and all tumor masses removed. For the flow cytometry analysis of tumour-infiltrating lymphocytes, tumours were minced, B16 tumor masses, collected at experimental endpoint, were digested for 1 hour with Collagenase D (10 mg/ml) and DNAseI (10 µg/ml) whereas 4T1 tumours were processed with Miltenyi dissociation kit (130-096-730 ) according to manufacturer's instructions.

1-2 × 10⁶ cells per sample were stained with the LIVE/DEAD stain (Invitrogen L34959), CD45, CD3, CD8, CD4, CD44, FOXP3 and CD45 followed by fixation with formaldehyde. Data acquisition was performed on Attune NxT Flow Cytometer. Results were analysed with the FlowJo software.

### (4) Immunotherapeutic agents used in the in vivo experiments.

In the above-described experimental procedures, the antibodies reported in Table 5 were used. The Catalogue number is from the antibody catalogue by BioXCell company, available at: https://bxcell.com/shop-products/

**Table 5. Agents used in the in vivo experiments.**

| **Name** | **Antigen** | **Clone** | **Catalogue No.** |
|---|---|---|---|
| InVivoMAb antimouse PD-1 (CD279) | PD-1 (CD279) | J43 | BE0033-2 |
| InVivoMAb antimouse PD-L1 (B7H1) | PD-L1 | 10F.9G2 | BE0101 |
| InVivoMAb antimouse CTLA-4 (CD152) | CTLA-4 (CD152) | UC10-4F10-11 | BE0032 |
| InVivoMAb anti-mouse OX-40 (CD134) | OX-40 (CD134) | OX-86 | BE0031 |
| InVivoPlus polyclonal antibody Armenian Hamster IgG | N/A | N/A | BE0091 |
| InVivoMAb anti-mouse CD8α | CD8 | 2.43 | BE0061 |

### (5) Standard diet and FMD

The mice were fed a standard diet (16% energy from proteins, 46% from carbohydrates, 38% from fat, total energy: 3.75 Kcal/g of food) or FMD throughout the course of the experiment. The FMD is a low-calorie diet based on vegetable compounds that is performed for 4 to 5 days. On the first day the mice are subjected to a calorie restriction diet that provides 50% of the daily energy requirement (5 g of "FMD day 1" diet as defined in Table 6, corresponding to 9.16 Kcal) determined according to the method described by Bachmanov, Behav Genet. 2002 Nov; 32(6): 435-443, while in the following three days the mice are fed a diet whose caloric intake is equal to 10% of the daily requirement (5 g of "FMD day 2-5" as defined in Table 6, corresponding to 1.80 Kcal). At the end of each FMD cycle mice are fed a standard diet for 3 days before starting a new FMD cycle. The number of FMD cycles vary according to the experimental conditions. In experiment of figure 1 there are 3 cycles of FMD, in experiment of figures 2, 3 and 5 animals were subjected to two FMD cycles , while in experiment of figure 4 it is only 1 FMD cycle was performed. In experiment of Figure 7, 4 cycles of FMD were performed. Mice weight was monitored during all days of FMD and if the mice lost more than 20% of their body weight they were immediately fed with a standard diet.

**Table 6. Energy density of FMD diets administered to mice.**

| | |
|---|---|
| FMD day 1 | 1.83 Kcal/g of food (0.11 Kcal/g proteins; 0.53 Kcal/g carbohydrates; 1.19 Kcal/g fatty acids); |
| FMD day 2-5 | 0.36 Kcal/g of food (0.0023 Kcal/g proteins; 0.35 Kcal/g carbohydrates; 0.0023 Kcal/g fatty acids). |

### (6) Toxicity assessment

In this experiment inventors evaluate the cytotoxicity of immunotherapy (anti-PDL1 and anti-OX40) in BALB/c mice carrying 4T1 tumors. As reported in the experimental scheme of figure 7, 3 days after the injection of 4T1 cells into the mammary fat pad, the mice undergo 1 cycle of FMD (4 days) every week for 4 weeks and 3 treatments with IgG, anti-PD -L1 and/or anti-OX40 3 times a week on alternate days for 1 week. The mice belonging to anti-PD-L1/OX40 group are treated the first week 3 times with anti-OX40, the second week 3 times with anti-PD-L1, while the mice of the anti-PD-L1 and OX40 group do not receive any treatment the second week. Starting from the third week, the mice are treated with IgG, anti-PD-L1 or anti-OX40 1 time per week on the last day of FMD. Survival of mice over time was monitored.

### EXAMPLES

### EXAMPLE 1

The inventors investigated whether FMD could enhance the therapeutic activity of anti-PD-L1 in ICI (immune checkpoint inhibitor) or ICB (immune checkpoint blockade) resistant B16 melanoma. In the first set of experiments they injected subcutaneously B16F10 melanoma cells into C57Bl / 6 mice. When the tumour mass was palpable, mice underwent FMD cycles (reduced calory intake by 50 % day 1 and reduced calory intake by 90% days 2, 3 and 4) every week and were treated 1 time a week for 3 weeks with anti-PD-L1 (100 µg/mouse). Anti-PD-L1 monotherapy has no effect on tumour growth in standard diet fed mice, whereas FMD not only delay tumour growth but increases the anti-tumour effect of anti PD-L1 (Figure 1B). In fact, FMD results in significantly reduced tumor progression alone in the FMD IgG control group compared to the standard diet control group (AL IgG). Treatment with anti-PD-L1 in combination with FMD further reduces the volume and progression of tumor masses compared to the FMD IgG control group, while it has no effect on tumor growth in the group fed the standard diet. Thus, FMD alone is able to block tumor growth and increase the anti-tumor efficacy of anti-PD-L1.

The effect of FMD in inhibiting tumor progression is mediated by the immune system as the percentage of CD8 + T lymphocytes increases in the tumor bed. CD8 + T lymphocytes constitute the lymphocyte population that mediates the anti-tumor response.

Indeed, CD8+ T cell lymphocytes depletion achieved by administrating a specific anti-CD8 antibody into mice, blunts the antitumor effect of FMD in combination with anti-PD-L1 (Figure 1B, last lane) and the tumor masses in the group FMD anti-CD8 anti-PDL1 are larger than those in the FMD IgG group . This data demonstrated that the antitumor effect of FMD/anti-PD-L1 is immune mediated and CD8+ T cell dependent (Figure 1 B, last lane).

### EXAMPLE 2

The inventors next assess the effect of FMD and anti-PD-L1 treatment on tumour infiltrating lymphocytes which consist of a CD8 + and CD4 + T lymphocytes population that infiltrates the tumor bed and that regulates the anti-tumor immune response. Activated CD8⁺ T lymphocytes (CD8+ CD44+) recognize and attack cancer cells by releasing cytolytic enzymes, whereas CD4+ T lymphocytes can differentiate into helper T lymphocytes and support the cytotoxic action of CD8 or can differentiate into Treg (CD4⁺ FOXP3⁺) T lymphocytes which carry out an immunosuppressive activity and promote tumor growth. Monotherapy with anti-PD-L1 slightly increases the tumour infiltrating CD8+ cells compared to control-IgG treated mice (figure 1 C) but does not have any impact on CD4+ FoxP3+ infiltrating Tregs (figure 1D). Strikingly, FMD alone or in combination with anti-PD-L1 further increase CD8⁺ T cells population, compared to the single treatment.

### EXAMPLE 3

The inventors evaluated whether FMD could potentiate the efficacy of anti-PDL1 and anti-PD1 in combination with anti-CTLA4. Mice were subcutaneously injected with 10⁵ B16F10 cells and subjected to 2 FMD cycles and treated with anti-PD1 or anti-PD-L1 (100 µg/mouse) and anti-CTLA4 every 2 days. FMD alone and in combination with anti PD1/CTLA4 and anti-PD-L1/CTLA4 was more effective in delaying tumour growth (volume and weight) compared to mice fed with standard diet and treated with anti-PD1/CTLA4 or anti-PD-L1/CTLA4. The inventors also confirm that the antitumor effect of FMD on tumour growth is immune mediated. Indeed the depletion of CD8+ T cell lymphocytes by administering specific anti-CD8 antibody into mice, reverts the anti-tumor effect of FMD on tumor growth (figure 2B, 2C).

FMD in combination with the anti-PD1/CTLA4 or anti-PD-L1/CTLA4 treatment improves the anti-tumor efficacy of the single anti-PD-L1 treatment. Anti-PD1/CTLA4 and anti-PD-L1/ CTLA4 has minimal marginal effect on tumor growth in the standard diet fed mice group. The inventors also evaluate whether only 2 cycles of FMD in combination with higher dose of immunotherapies are able to recapitulate the same effects observed in Fig. 2.

In the experiment of figure 3, mice were treated intraperitoneally (i.p.) with anti-PD-1 antibody (at the dose of 250 µg per mouse), anti-CTLA4 antibody (at dose of 200 µg per mouse the first injection and 100 µg per mouse the second and third injection) and IgG 450 µg per mouse. The immunotherapeutics or IgG were administered only during week 1 (first cycle of FMD). Mice belonging to FMD group underwent 2 cycles of FMD, once per week.

Since the inventors administered the immunotherapeutics only during week 1, they increased the dose.

### EXAMPLE 4 (Reference example)

Inventors tested whether 1 cycle of FMD in combination with anti-PD1 (250 µg/mouse), anti-CTLA-4 (200 µg/mouse 1st treatment, 100 µg/mouse 2nd and 3rd treatment), administered every other day three times in 1 week, could have the same beneficial effect on tumor progression in B16F10 tumor-bearing mice.

The anti-PD1/CTLA-4 combined therapy exerted anti-tumour effect against B16F10 tumour in mice subjected to only 1 cycle of FMD (figure 4). Inventors did not observe any anti-tumour response to anti-PD1/CTLA-4 therapy in mice fed with standard diet. It is worth to note that 2 FMD cycles were able to decrease tumour size (volume and weight, figure 3), whereas 1 FMD cycle does not affect tumour growth compared to mice fed with standard diet (figure 4).

### EXAMPLE 5

Then the inventors tested the efficacy of FMD in combination with anti-PDL1 and anti-OX40 against triple negative 4T1 breast cancer. BALB/c mice were injected with 5x10⁴ 4T1 cell in mammary fat pad and treated with anti-PD-L1 (100 µg/mouse) or anti-OX40 (100 µg/mouse) or both three times per week every other day for 1 week in the single therapy group and for 2 weeks in combined treatment. The combined anti-PDL1 and anti-OX40 treatment was administered not concurrently but sequentially: mice were treated with anti-OX40 1st week, whereas with anti-PD-L1 the 2nd week. The anti-PD-L1 and anti-OX40 monotherapy are ineffective at treating 4T1 breast cancer in BALB/c mice fed with standard diet. FMD in combination with anti-PD-L1 or OX40 reduces 4T1 tumour size whereas it elicits an additive effect in tumour volume and weight reduction when used in combination with anti-PD-L1 and anti-OX40 (figure 5 B and C). FMD prevent splenomegaly specially in mice treated with the combination of anti-PD-L1 and OX40 (Figure 5D). The spleen enlargement is generally due to an inflammatory state characterized by an increase in the red pulp, consisting mainly of red and white blood cells, and a strong reduction in the white pulp, an area of the spleen rich in B and T lymphocytes important for the adaptive immune response. In mice subjected to FMD the white pulp is more preserved and has multiple germinal centers, which indicate that splenic functionality is not compromised as in mice fed with a standard diet.

Analysis of tumor infiltrating lymphocytes (TILs) showed that the percentage of total CD3⁺ T lymphocytes (Figure 6a) and of CD4⁺ T lymphocytes subpopulation (Figure 6b) does not change in the various experimental conditions (standard diet or FMD; anti-PD- L1 and anti-OX40). On the other hand, FMD in combination with anti-PD-L1 increases the percentage of CD8 ⁺ cytotoxic T lymphocytes (figure 6c) while it does not affect CD8⁺ CD44⁺ Cd25⁻T cells activation (figure 6d). In the anti-PD-L1 and anti-OX40 FMD group the population of infiltrating CD8⁺ T lymphocytes in the tumor bed does not change compared to the FMD/IgG controls (Figure 6c), however a higher percentage of CD8⁺CD44⁺CD25⁻ active T lymphocytes is observed, which could explain the antitumoral effect observed on tumor masses growth in this group.

Analysis of tumour infiltrating lymphocytes showed that FMD plus anti-PD-L1 or anti-OX40 or both increase the percentage of CD8+ T cell in tumour bed, whereas FMD in combination with anti-PD-L1/OX40 promotes CD44⁺CD8⁺ T cell lymphocytes activation (Figure 6). Unexpectedly the inventors noticed that if they treat 4T1 tumor bearing mice with PD-L1 and anti OX40 in combination with FMD, on the 3rd and 4th week, 1 time per week, they observe that the anti-PD-L1 and anti-OX40 treatment on the 3^{rd} week result to be highly toxic for the animals fed with standard diet and cause suddenly death within 15'-30' upon ICI injection. Furthermore, the anti-PDL1 therapy was lethal already the 2^{nd} week of treatment in some mice belonging to anti-PD-L1/OX40 combined treatment group (figure 7).

In other words, mice fed with standard diet are not very tolerant to the treatment with anti-PD-L1 and/or anti-OX40 already starting from the second week, while mice fed with FMD tolerate very well the treatment. Single anti-PD-L1 or anti-OX40 treatment is lethal to mice belonging to standard diet groups from the third week, while it has no detrimental effect in the groups fed with FMD.

As shown in figure 7, FMD (4 cycles separated by 3 days of food ad libitum) protects mice from anti-PD-L1 and OX40 induced-toxicity starting from the 3 week of treatment and boosts the antitumor efficacy of ICB therapy.

In patients, FMD regimen, may consist in a 5-day regimen, calorie-restricted (up to 1100 Kcal administered on day 1; up to 750 Kcal administered on days 2, 3, 4, 5), low-carbohydrate, low-protein diet.

In patients, FMD regimen, may also consist in a 4-days, calorie-restricted (up to 1100 Kcal administered on day 1; up to 750 Kcal administered on days 2, 3, 4), low-carbohydrate, low-protein diet.

For clinical trials on breast cancer or melanoma patients a FMD diet consisting of a 5-days, calorie-restricted (up to 600 Kcal administered on day 1; up to 300 Kcal administered on days 2, 3, 4, 5), low-carbohydrate, low -protein diet is adopted. FMD is repeated every 28 days for at least 2 times and immunotherapy is given on the third day of FMD. Immunotherapy is administered according to the doses reported in clinical protocol.

Breast cancer or melanoma patients can follow FMD diet consisting of a 5-days, calorie-restricted (up to 600 Kcal administered on day 1; up to 300 Kcal administered on days 2, 3, 4, 5), low-carbohydrate, low -protein diet, that can be repeated every 28 days for at least 2 times while immunotherapy is given on the third day of FMD. Immunotherapeutic agents are administered according to the doses known to the person with ordinary skill in the art.

### REFERENCES

- 1.: Bianchi G, et al. Oncotarget. 2015 May 20;6(14):11806-19.
- 2.: Bulliard Y, et al., Immunol Cell Biol. 2014;92(6):475-480.
- 3.: Calabro L, et al., Lancet Oncol. 2013; 14:1104-11.
- 4.: Curti BD, et al. Cancer Res. 2013;73(24):7189-7198.
- 5.: Di Biase S, et al. Cancer Cell. 2016 Jul 11;30(1):136-146.
- 6.: Dougall WC, et al., Immunol Rev. 2017;276(1):112-120.
- 7.: El-Khoueiry AB et al. Lancet 389, 2492-2502 (2017).
- 8.: Esensten JH, et al., Immunity. 2016; 44(5):973-988.
- 9.: Foley EJ Cancer Res 13, 835-837 (1953).
- 10.: Fourcade J et al. J. Exp. Med 207, 2175-2186 (2010).
- 11.: Garon EB et al. N. Engl. J. Med 372, 2018-2028 (2015).
- 12.: Kim ST et al. Nat. Med 24, 1449-1458 (2018). [PubMed: 30013197]
- 13.: Larkin J, et al., N Engl J Med. (2015) 373:1270-1. doi: 10.1056/NEJMc1509660
- 14.: Lesokhin AM, et al., J. of Clinical Oncology, vol. 34, no. 23, pp. 2698-2704, 2016.
- 15.: Lee C, et al. Science Translational Medicine. 2012;4(124):124ra27.
- 16.: Lee C, Oncogene. 2011;30(30):3305-16. doi: 10.1038/onc.2011.91.
- 17.: Levine ME, et al. Cell Metabolism. 2014;19(3):407-17.
- 18.: Longo VD, Science 2003;299(5611):1342-6.
- 19.: Matsuzaki J et al. Proc. Natl Acad. Sci. USA 107, 7875-7880 (2010).
- 20.: Montler R, et al.. Clin Transl Immunol. (2016) 5:e70. 10.1038/cti.2016.16
- 21.: Motzer RJ et al. J. Clin. Oncol 33, 1430-1437 (2015).
- 22.: Mueller DL, Jenkins MK, Schwartz RH. Annu Rev Immunol. 1989;7:445-480.
- 23.: Piconese S, Valzasina B, Colombo MP. J Exp Med. 2008;205(6):1505].
- 24.: Postow MA, et al. N Engl J Med. (2015) 372:2006-17. doi: 10.1056/NEJMoa1414428
- 25.: Raffaghello L, et al. PNAS 2008; 105(24):8215-20.
- 26.: Ribas A, Hamid O, Daud A, Hodi FS, et al. JAMA. 2016 Apr 19;315(15):1600-9.
- 27.: Ribas A & Wolchok JD Science 359, 1350-1355 (2018).
- 28.: Robert C, et al. N Engl J Med. 2015 Jan 22;372(4):320-30.
- 29.: Robert C, et al. N Engl J Med. 2015 Jun 25;372(26):2521-32.
- 30.: Rosenberg JE et al. Lancet 387, 1909-1920 (2016).
- 31.: Sade-Feldman M., et al., Nat Commun. 2017 Oct 26;8(1):1136.
- 32.: Sangro B, et al. JHepatol.2013;59:81-8.
- 33.: Sarnaik, A.A. et al. Clin. Cancer Res. 17, 896-906 (2011).
- 34.: Schadendorf D, et al. J Clin Oncol. (2015) 33:1889-94.
- 35.: Shim HS, Wei M, Brandhorst S, Longo VD. Cancer Res. 2015 Mar 15;75(6):1056-67
- 36.: Slovin SF, et al. AnnOncol.2013;24:1813-21.
- 37.: Smith HJ Br. J. Cancer 20, 831-837 (1966).
- 38.: Topalian SL et al. N. Engl. J. Med 366, 2443-2454 (2012).
- 39.: Ward-Kavanagh LK, Lin WW, Sedy' JR, Ware CF. Immunity. 2016;44(5):1005-1019.
- 40.: Walunas TL et al. Immunity 1, 405-413 (1994).
- 41.: Weinberg AD, Rivera MM, Prell R, et al. J Immunol. 2000;164(4):2160-2169.
- 42.: Wikenheiser DJ et al., Front Immunol. 2016; 7:304.
- 43.: Wolchok JD, et al. N Engl J Med 2017; 377: 1345-1356.
- 44.: Wolchok, J.D. et al. Lancet Oncol. 11, 155-164 (2010).
- 45.: Yang JC, et al. JImmunother. 2007; 30:825-30.

## Claims

1. At least one reduced caloric intake cycle and at least one PD-L1 inhibitor for use in the treatment of cancer wherein said at least one reduced caloric intake cycle comprises a first part with a regular caloric intake reduced by 30% to 70% and a second part with a regular caloric intake reduced by 40 to 97% and wherein said at least one PD-L1 inhibitor is an anti-PDL1 antibody.

2. The at least one reduced caloric intake cycle and at least one PD-L1 inhibitor for use according to claim 1, wherein said first part and/or said second part lasts for a period of 24 to 190 hours, preferably said first part and/or or said second part lasts for a period of 24 to 120 hours, preferably said first part and/or or said second part lasts for approximately 120 hours.

3. The at least one reduced caloric intake cycle and at least one PD-L1 inhibitor for use according to claim 1 or 2, wherein the at least one reduced caloric intake cycle is repeated from 1 to 30 times after respective periods of from 5 to 60 days.

4. The at least one reduced caloric intake cycle and at least one PD-L1 inhibitor for use according to any one of previous claims, wherein the PD-L1 inhibitor is selected from the group consisting of: atezolizumab, durvalumab, avelumab, APL-502, bintrafusp alfa, CS-1001, KN-035, SHR-1316, BGBA-333, CX-072, GEN-1046, GS-4224, KD-005, KLA-167, KN-046, STIA-1014, ADG-104, AK-106, BCD-135, cosibelimab, FAZ-053, FPT-155, FS-118, HLX-20, IBI-318, INBRX-105, JS-003, lodapolimab, LP-002, LY-3434172, MCLA-145, MSB-2311, SHR-1701, SL-279252, STIA-1015, or a combination thereof.

5. The at least one reduced caloric intake cycle and at least one PD-L1 inhibitor for use according to claim 4, wherein the PD-L1 inhibitor is atezolizumab.

6. The at least one reduced caloric intake cycle and at least one PD-L1 inhibitor for use according to anyone of previous claims, comprising administering a combination of:
- PD-L1 inhibitor and CTLA-4 inhibitor; or
- PD-L1 inhibitor and OX40 activator.

7. The at least one reduced caloric intake cycle and at least one PD-L1 inhibitor for use according to any one of previous claims, comprising administering a further therapeutic intervention, preferably said further therapeutic intervention is selected from the group consisting of: surgery, radiotherapy and at least one further therapeutic agent, preferably said further therapeutic agent is a further immune checkpoint inhibitor, an immune response stimulator, a targeted anticancer agent, a DNA Damage Response inhibitor and/or a chemotherapeutic agent, preferably said further immune checkpoint inhibitor is selected from the group consisting of: PD1 inhibitors, PDL1 inhibitors, CTLA-4 inhibitors, TIGIT inhibitors, ICOS inhibitors, TIM3 inhibitors, IDO1 inhibitors; said immune response stimulator is selected from the group consisting of: OX40 activators, GITR modulators, 4-1BB agonists; said targeted anticancer agent is selected from the group consisting of: PI3K inhibitors, HDAC inhibitors, EGFR inhibitors, BRAF inhibitors, MAPK inhibitors, CDK inhibitors, ER stress activators; said DNA Damage Response inhibitor is selected from the group consisting of: PARP inhibitors, CHK1 inhibitors, ATR inhibitors, Wee1 inhibitors; said chemotherapeutic agent is selected from the group consisting of: Alkylating agents, Antimetabolites, Anti-microtubule agents, Topoisomerase inhibitors, Cytotoxic antibiotics.

8. The at least one reduced caloric intake cycle and at least one PD-L1 inhibitor for use according to any one of previous claims, wherein said cancer is **characterized by** resistance or partial response to the treatment with at least one immunotherapeutic agent.

9. The at least one reduced caloric intake cycle and at least one PD-L1 inhibitor for use according to any one of previous claims, wherein said cancer is a solid or hematopoietic cancer, preferably the cancer is selected from the group consisting of: breast cancer, melanoma, lymphoma, lung cancer, non-small cell lung cancer (NSCLC), head and neck cancer, gastroesophageal cancer, bladder cancer and urothelial cancer, hepatocellular carcinoma and renal cell carcinoma.

## Patentansprüche

1. Mindestens ein Zyklus mit reduzierter Kalorienzufuhr und mindestens ein PD-L1-Inhibitor zur Verwendung bei der Behandlung von Krebs, wobei der mindestens eine Zyklus mit reduzierter Kalorienzufuhr einen ersten Teil mit einer regulären Kalorienaufnahme, die um 30 % bis 70 % reduziert ist, und einen zweiten Teil mit einer regulären Kalorienaufnahme, die um 40 % bis 97 % reduziert ist, umfasst, und wobei der mindestens eine PD-L1-Inhibitor ein Anti-PD-L1-Antikörper ist.

2. Der mindestens eine Zyklus mit reduzierter Kalorienzufuhr und mindestens eine PD-L1-Inhibitor zur Verwendung gemäß Anspruch 1, wobei der erste Teil und/oder der zweite Teil für einen Zeitraum von 24 bis 190 Stunden andauert, bevorzugt der erste Teil und/oder der zweite Teil für einen Zeitraum von 24 bis 120 Stunden andauern, bevorzugt der erste Teil und/oder der zweite Teil für annähernd 120 Stunden andauern.

3. Der mindestens eine Zyklus mit reduzierter Kalorienzufuhr und mindestens eine PD-L1-Inhibitor zur Verwendung gemäß Anspruch 1 oder 2, wobei der mindestens eine Zyklus mit reduzierter Kalorienzufuhr 1 bis 30 Mal nach jeweiligen Zeiträumen von 5 bis 60 Tagen wiederholt wird.

4. . Der mindestens eine Zyklus mit reduzierter Kalorienzufuhr und mindestens eine PD-L1-Inhibitor zur Verwendung gemäß einem der vorangehenden Ansprüche, wobei der PD-L1-Inhibitor ausgewählt ist aus der Gruppe bestehend aus Atezolizumab, Durvalumab, Avelumab, APL-502, Bintrafusp alfa, CS-1001, KN-035, SHR-1316, BGBA-333, CX-072, GEN-1046, GS-4224, KD-005, KLA-167, KN-046, STIA-1014, ADG-104, AK-106, BCD-135, Cosibelimab, FAZ-053, FPT-155, FS-118, HLX-20, IBI-318, INBRX-105, JS-003, Jodapolimab, LP-002, LY-3434172, MCLA-145, MSB-2311, SHR-1701, SL-279252, STIA-1015, oder einer Kombination davon.

5. Der mindestens eine Zyklus mit reduzierter Kalorienzufuhr und mindestens eine PD-L1-Inhibitor zur Verwendung gemäß Anspruch 4, wobei der PD-L1-Inhibitor Atezolizumab ist.

6. Der mindestens eine Zyklus mit reduzierter Kalorienzufuhr und mindestens eine PD-L1-Inhibitor zur Verwendung gemäß einem der vorangehenden Ansprüche, umfassend das Verabreichen einer Kombination aus:
- PD-L1-Inhibitor und CTLA-4-Inhibitor; oder
- PD-L1-Inhibitor und OX40-Aktivator.

7. Der mindestens eine Zyklus mit reduzierter Kalorienzufuhr und mindestens eine PD-L1-Inhibitor zur Verwendung gemäß einem der vorangehenden Ansprüche, umfassend das Verabreichen einer weiteren therapeutischen Intervention, vorzugsweise wobei diese weitere therapeutische Intervention ausgewählt ist aus der Gruppe bestehend aus Chirurgie, Radiotherapie und mindestens einem weiteren therapeutischen Mittel, vorzugsweise wobei dieses weitere therapeutische Mittel ein weiterer Immun-Checkpoint-Inhibitor, ein Immunantwort-Stimulator, ein zielgerichteter Antikrebswirkstoff, ein DNA-Schadensreaktions-Inhibitor und/oder ein chemotherapeutisches Mittel ist, vorzugsweise wobei der weitere Immun-Checkpoint-Inhibitor ausgewählt ist aus der Gruppe bestehend aus: PD1-Inhibitoren, PD-L1-Inhibitoren, CTLA-4-Inhibitoren, TIGIT-Inhibitoren, ICOS-Inhibitoren, TIM3-Inhibitoren, IDO1-Inhibitoren; der Immunantwort-Stimulator ausgewählt ist aus der Gruppe bestehend aus: OX40-Aktivatoren, GITR-Modulatoren, 4-IBB-Agonisten; der zielgerichtete Antikrebswirkstoff ausgewählt ist aus der Gruppe bestehend aus: PI3K-Inhibitoren, HDAC-Inhibitoren, EGFR-Inhibitoren, BRAF-Inhibitoren, MAPK-Inhibitoren, CDK-Inhibitoren, ER-Stress-Aktivatoren; der DNA-Schadensreaktions-Inhibitor ausgewählt ist aus der Gruppe bestehend aus: PARP-Inhibitoren, CHK1-Inhibitoren, ATR-Inhibitoren, Wee1-Inhibitoren; das chemotherapeutische Mittel ausgewählt ist aus der Gruppe bestehend aus: Alkylierungsmitteln, Antimetaboliten, Anti-Mikrotubuli-Wirkstoffen, Topoisomerase-Hemmern, cytotoxischen Antibiotika.

8. Der mindestens eine Zyklus mit reduzierter Kalorienzufuhr und mindestens eine PD-L1-Inhibitor zur Verwendung gemäß einem der vorangehenden Ansprüche, wobei der Krebs durch Resistenz oder teilweises Ansprechen auf die Behandlung mit mindestens einem immuntherapeutischen Mittel gekennzeichnet ist.

9. Der mindestens eine Zyklus mit reduzierter Kalorienzufuhr und mindestens eine PD-L1-Inhibitor zur Verwendung gemäß einem der vorangehenden Ansprüche, wobei der Krebs ein solider oder hämatopoetischer Krebs ist, vorzugsweise wobei der Krebs ausgewählt ist aus der Gruppe bestehend aus: Brustkrebs, Melanom, Lymphom, Lungenkrebs, nicht-kleinzelliger Lungenkrebs (NSCLC), Kopf- und Halskrebs, Magen- und Speiseröhrenkrebs, Blasenkrebs und Urothelkarzinom, Leberzellkarzinom und Nierenzellkarzinom.

## Revendications

1. Au moins un cycle d'absorption calorique réduite et au moins un inhibiteur de PD-L1 destinés à être utilisés dans le traitement du cancer dans lesquels ledit au moins un cycle d'absorption calorique réduite comprend une première partie avec une absorption calorique régulière réduite de 30 % à 70 % et une seconde partie avec une absorption calorique régulière réduite de 40 % à 97 % et ledit au moins un inhibiteur de PD-L1 étant un anticorps anti-PD-L1.

2. L'au moins un cycle d'absorption calorique réduite et au moins un inhibiteur de PD-L1 destinés à être utilisés selon la revendication 1,
dans lesquels la dite première partie et/ou ladite seconde partie
dure durant un période de 24 à 190 heures, de préférence ladite première partie et/ou ladite seconde partie dure durant une période de 24 à 120 heures, de préférence ladite première partie et/ou ladite seconde partie dure durant environ 120 heures.

3. L'au moins un cycle d'absorption calorique réduite et au moins un inhibiteur de PD-L1 destinés à être utilisés selon la revendication 1 ou 2, dans lesquels l'au moins un cycle d'absorption calorique réduite est répété de 1 à 30 fois après des périodes respectives de 5 à 60 jours.

4. L'au moins un cycle d'absorption calorique réduite et au moins un inhibiteur de PD-L1 destinés à être utilisés selon l'une quelconque des revendications précédentes, dans lesquels l'inhibiteur de PD-L1 est choisi dans le groupe constitué par l'atézolizumab, le durvalumab, l'avélumab, APL-502, le bintrafusp alfa, CS-1001, KN-035, SHR-1316, BGBA-333, CX-072, GEN-1046, GS-4224, KD-005, KLA-167, KN-046, STIA-1014, ADG-104, AK-106, BCD-135, le cosibélimab, FAZ-053, FPT-155, FS-118, HLX-20, IBI-318, INBRX-105, JS-003, le lodapolimab, LP-002, LY-3434172, MCLA-145, MSB-2311, SHR-1701, SI-279252, STIA-1015 ou une de leurs combinaisons.

5. L'au moins un cycle d'absorption calorique réduite et au moins un inhibiteur de PD-L1 destinés à être utilisés selon la revendication 4, dans lesquels l'inhibiteur de PD-L1 est l'atézolizumab.

6. L'au moins un cycle d'absorption calorique réduite et au moins un inhibiteur de PD-L1 destinés à être utilisés selon l'une quelconque des revendications précédentes, comprenant l'administration d'une combinnaison de
- inhibiteur de PD-L1 et inhibiteur de CTLA-4 ou
- inhibiteur de PD-L1et activateur d'OX40.

7. L'au moins un cycle d'absorption calorique réduite et au moins un inhibiteur de PD-L1 destinés à être utilisés selon l'une quelconque des revendications précédentes, comprenant l'administration d'une autre intervention thérapeutique, de préférence ladite autre intervention thérapeutique est choisie dans le groupe constitué par la chirurgie, la radiothérapie et au moins un autre agent thérapeutique, de préférence ledit autre agent thérapeutique est un autre inhibiteur de point de contrôle immunitaire, un stimulateur de réponse immunitaire, un agent anticancéreux ciblé, un inhibiteur de réponse de dommage à l'ADN, de préférence ledit autre inhibiteur de point de contrôle immunitaire est choisi dans le groupe constitué par les inhibiteurs de PD1, les inhibiteurs de PDL1, les inhibiteurs de CTLA-4, les inhibiteurs de TIGIT, les inhibiteurs d'ICOS, les inhibiteurs de TIM3, les inhibiteurs d'IDO1, ledit stimulateur de réponse immunitaire est choisi dan le groupe constitué par les activateurs d'OX40, le modulateurs de GITR, les agonistes d'IBB, ledit agent anticancéreux ciblé est choisi dans le groupe constitué par les inhibiteurs de PI3K, les inhibiteurs de HDAC, les inhibiteurs de EGFR, les inhibiteurs de BRAF, les inhibiteurs de MAPK, les inhibiteurs de CDK, les activateurs de stress ER, ledit inhibiteur de réponse au dommage à l'ADN est chois dans le groupe constitué par les inhibiteurs de PARP, les inhibiteurs de CHK1, les inhibiteurs d'ATR, les inhibiteurs de Wee1, ledit agent chimiothérapeutique est choisi dans le groupe constitué par les agents alkylants, les antimétabolites, les agents anti-microtubule, les inhibiteurs de topoisomérase, les antibiotiques cytotoxiques.

8. L'au moins un cycle d'absorption calorique réduite et au moins un inhibiteur de PD-L1 destinés à être utilisés selon l'une quelconque des revendications précédentes, dans lequel ledit cancer est **caractérisé par** la résistance ou une réponse partielle au traitement avec au moins un agent chimiothérapeutique.

9. L'au moins un cycle d'absorption calorique réduite et au moins un inhibiteur de PD-L1 destinés à être utilisés selon l'une quelconque des revendications précédentes, dans lesquels ledit cancer est un cancer solide ou hématopoïétique, de préférence le cancer est choisi dans le groupe constitué par le cancer du sein, le mélanome, le lymphome, le cancer du poumon, le cancer du poumon à non petite cellule (NSCLC), le cancer de la tête et du cou, le cancer gastroœsophagien, le cancer de la vessie et le cancer urothélial, le carcinome - hépatocellulaire et le carcinome de cellule rénale.
